Europäisches Patentamt

European Patent Office

Office européen des brevets

(11) Veröffentlichungsnummer: **0 400 609**
**A1**

## (12) EUROPÄISCHE PATENTANMELDUNG

(21) Anmeldenummer: 90110267.3

(22) Anmeldetag: 30.05.90

(51) Int. Cl.5: **A61K 31/55, A61K 9/18,**
**A61K 47/40, A61K 47/48**

(30) Priorität: 02.06.89 CH 2084/89

(43) Veröffentlichungstag der Anmeldung:
05.12.90 Patentblatt 90/49

(84) Benannte Vertragsstaaten:
AT BE CH DE DK ES FR GB GR IT LI LU NL SE

(71) Anmelder: CIBA-GEIGY AG
Klybeckstrasse 141
CH-4002 Basel(CH)

(72) Erfinder: Steulet, Anne-Françoise
Vernes 103
CH-2842 Rossemaison(CH)
Erfinder: Schmutz, Markus, Dr.
Baumgartenweg 24
CH-4124 Schönenbuch(CH)
Erfinder: Maître, Laurent, Dr.
Pfeffingerstrasse 99
CH-4053 Basel(CH)
Erfinder: Bernasconi, Raymond, Dr.
Amselstrasse 17A
CH-4104 Oberwil(CH)
Erfinder: Stahl, Peter Heinrich, Dr.
Lerchenstrasse 28
D-7800 Freiburg(DE)

(74) Vertreter: Zumstein, Fritz, Dr. et al
Bräuhausstrasse 4
D-8000 München 2(DE)

(54) Intravenöse Lösungen mit schnellem Wirkungseintritt.

(57) Die Erfindung betrifft eine pharmazeutische Zusammensetzung für die intravenöse Applikation von Carbamazepin mit schnellem Wirkungseintritt. Die Zusammensetzung enthält:
    a) Carbamazepin;
    b) als Löslichkeitsvermittler durch $C_1$-$C_4$-Alkyl und/oder Hydroxy-$C_2$-$C_4$-alkyl veräthertes $\beta$-Cyclodextrin und
    c) die Trägerflüssigkeit und gegebenenfalls weitere wasserlösliche pharmazeutische Hilfsstoffe.

EP 0 400 609 A1

## Intravenöse Lösungen mit schnellem Wirkungseintritt

Die Erfindung hat eine pharmazeutische Zusammensetzung für die intravenöse Applikation von Carbamazepin zum Gegenstand, das Trockenpräparat enthaltend Carbamazepin und einen Löslichkeitsvermittler, Verfahren zur Herstellung der intravenös applizierbaren pharmazeutischen Zusammensetzung und des Trockenpräparats sowie die Anwendung der pharmazeutischen Zusammensetzung in einem therapeutischen Verfahren, insbesondere als Antineuralgikum und Antikonvulsivum mit schnellem Wirkungseintritt.

Carbamazepin, 5H-Dibenz[b,f]azepin-5-carboxamid (Tegretol®, Tegretal®: Ciba-Geigy), gilt als Mittel der ersten Wahl bei der Behandlung von Konvulsionen und starken Schmerzzuständen. Die handelsüblichen Darreichungsformen sind für die perorale Verabreichung vorgesehen, z.B. Tabletten mit 200 mg Wirkstoff oder Sirupe mit 2%-igem Wirkstoffgehalt. Diese Darreichungsformen eignen sich insbesondere für regelmässig wiederkehrende Verabreichungen in einem längeren Behandlungszeitraum, um unter "normalen" Bedingungen eine gleichmässige Wirkstoffkonzentration im Blut sicherzustellen.

Bei akuten epileptischen Anfällen verbunden mit lebensgefährdenden krampfartigen Zuständen, z.B. dem Status epilepticus, welche Sofortapplikationen mit möglichst raschem Wirkungseintritt zur Beendigung der Krämpfe unbedingt erfordern, sind die oralen Darreichungsformen mit ihrem relativ langsamen Wirkungseintritt ungeeignet. Diese können - im Gegensatz zu intravenösen Lösungen - während des Krampfanfalls gar nicht appliziert werden. Dasselbe Problem tritt bei sich intensivierenden, lang anhaltenden Schmerzzuständen auf.

Der vorliegenden Erfindung liegt die Aufgabe zugrunde, für den bewährten Wirkstoff Carbamazepin eine neue Darreichungsform mit schnellem Wirkungseintritt bereitzustellen, welche für die genannten krampfartigen Zustände unmittelbar nach ihrem Auftreten verwendbar ist. Der schnelle Wirkungseintritt macht diese Darreichungsform insbesondere für die Notfallmedizin geeignet.

Schneller Wirkungseintritt lässt sich insbesondere durch Verwendung von intravenös applizierbaren Darreichungsformen mit gelösten Wirkstoffen erzielen, z.B. Injektions-oder Infusionslösungen. Wegen seiner geringen Wasserlöslichkeit ist Carbamazepin für diese Darreichungsformen ungeeignet, da die therapeutisch erforderliche Mindestdosis dieses Wirkstoffs in Wasser nicht in gelöstem Zustand, sondern nur in Form einer Suspension vorliegen kann, deren intravenöse Applikation unzulässig ist.

Zur Verbesserung der Löslichkeit für parenterale Zwecke sind für verschiedene schwerlösliche Wirkstoffe Löslichkeitsvermittler bekannt, z.B. hydrophile Cosolventien wie Ethanol, Glycerin, Propylenglycol, flüssige Polyethylenglycole oder lipophile Löslichkeitsvermittler wie Lecithin, Fettsäurepolyglycolester oder Fettsäureglycerinpolyglycolester. Für Carbamazepin ist kein spezieller' Löslichkeitsvermittler zugelassen worden. Die Verwendung von Löslichkeitsvermittlern ist wegen mangelnder Verträglichkeit und Stabilität, z.B. Entmischungseffekten bei Lagerung der parenteralen Suspension, generell problematisch.

Man hat daher vorgeschlagen, die Löslichkeit von schwerlöslichen Wirkstoffen, wie z.B. Pirprofen, durch Herstellung von löslichen Einschlussverbindungen mit Cyclodextrinen, z.B. $\alpha$-, $\beta$- oder $\gamma$-Cyclodextrin, zu erhöhen, siehe z.B. Deutsche Offenlegungsschrift 33 25 615.

Eine weitere Steigerung der Löslichkeit einiger schwerlöslicher Wirkstoffe lässt sich durch Herstellung von löslichen Einschlussverbindungen mit verätherten Cyclodextrinderivaten, z.B. gemäss dem in der U.S. Patentschrift 4,727, 064 beschriebenen Verfahren, erzielen. In der Internationalen Anmeldung WO 85/02767 sind u.a. parenteral applizierbare Darreichungsformen für diverse schwerlösliche Wirkstoffe eingeschlossen in verätherten $\beta$-Cyclodextrinderivaten beschrieben.

Die Aufgabenstellung, intravenös applizierbare Injektions- oder Infusionslösungen des schwerlöslichen Wirkstoffs Carbamazepin herzustellen, entzog sich bislang einer überzeugenden Durchführung, da bekannt war, dass wasserfreies Carbamazepin (amorph oder kristallin) in Kontakt mit Wasser das Dihydrat bildet, siehe J. Pharm. Soc. Jpn. No. 2, 184-190 (1984). Dieses Dihydrat liegt kristallin in Form von Nadeln vor, welche bis zu einer Teilchengrösse von ca. 100-500 μm und grösser anwachsen können. Diese Eigenschaft ist für parenterale Darreichungsformen besonders nachteilig, da die Bildung solcher Kristalle parenterale Lösungen unbrauchbar macht. Kristallsuspensionen dürfen intravenös nicht appliziert werden.

Es wurde überraschenderweise gefunden, dass bei Verwendung von verätherten $\beta$-Cyclodextrinderivaten, insbesondere Hydroxypropyl-$\beta$-cyclodextrin, kein Kristallwachstum von Hydratformen des Carbamazepins zu beobachten ist und dabei der Wirkstoff in einer für die parenterale Applikation geeigneten therapeutischen Menge in Wasser sich lösen oder zumindest dispergieren lässt.

Die vorliegende Erfindung betrifft eine pharmazeutische Zusammensetzung für die intravenöse Applikation von Carbamazepin enthaltend:

a) Carbamazepin;

b) als Löslichkeitsvermittler durch $C_1$-$C_4$-Alkyl und/oder Hydroxy-$C_2$-$C_4$-alkyl veräthertes $\beta$-Cyclodex-

trin, wobei die Hydroxygruppe sich in einer höheren als der 1-Stellung des $C_2$-$C_4$-Alkylrests befindet, und

c) die wässrige Trägerfiüssigkeit und gegebenenfalls weitere wasserlösliche pharmazeutische Hilfsstoffe.

Diese pharmazeutische Zusammensetzung zeichnet sich durch besondere Stabilität aus. So wird in einem Zeitraum bis zu mehreren Monaten keine Sedimentation, z.B. von Carbamazepinkristallen und/oder Hilfsstoffpartikeln, beobachtet. Die Zusammensetzung eignet sich ausserdem für die Herstellung einer intravenösen Darreichungsform mit einer therapeutisch wirksamen Dosis des schwerlöslichen Wirkstoffs Carbamazepin in einem relativ geringen Volumen, z.B. 2-10 ml, Trägerflüssigkeit.

Die weiter vorn und im folgenden verwendeten allgemeinen Definitionen und Begriffe haben im Rahmen der Beschreibung der Erfindung vorzugsweise die folgenden Bedeutungen:

Der Begriff pharmazeutische Zusammensetzung umfasst eine applikationsfähige Mischung, bestehend aus dem für die Verabreichung vorgesehenen Wirkstoff in jeweils therapeutischer Mindestmenge, der Trägerflüssigkeit und den für die betreffende Darreichungsform, z.B. Injektions- oder Infusionslösung, unter Berücksichtigung der Applikationsart - hier i.v. -, geeigneten Hilfsstoffen.

Der Begriff Löslichkeitsvermittler definiert einen Hilfsstoff oder ein Hilfsstoffgemisch, welches die Dispersionsfähigkeit eines in Wasser schwerlöslichen Wirkstoffs oder Wirkstoffgemischs soweit erhöht, dass therapeutisch wirksame Dosen des Wirkstoffs oder Wirkstoffgemisches gelöst oder zumindest kolloidal - im vorliegenden Fall i.v. - applizierbar sind. Der Begriff Dispersionsfähigkeit definiert ein Mass für die Bildung von echten molekularen Lösungen der Wirkstoffe und der Hilfsstoffe in Wasser sowie kolloidalen Lösungen, z.B. Lösungen aus Assoziationskolloiden oder Molekülkolloiden, die klar bzw. opaleszierend sind und gegebenenfalls nach Filtrieren, insbesondere mit sterilen Filtern mit ca. 5-10 μm Porendurchmesser, keinerlei Feststoffpartikel aufweisen, z.B. mizellare Lösungen oder Sphärokolloide, die nur in der Ultrazentrifuge abtrennbar sind. Die Dispersionsfähigkeit kann z.B. in mg oder mMol Wirkstoff pro Liter Wasser angegeben werden.

Durch $C_1$-$C_4$-Alkyl und/oder Hydroxy-$C_2$-$C_4$-alkyl veräthertes β-Cyclodextrin, wobei die Hydroxygruppe sich in einer höheren als der 1-Stellung des $C_2$-$C_4$-Alkylrests befindet, ist z.B. ein β-Cyclodextrinderivat mit 7 Anhydroglucoseeinheiten, welches an einer primären Hydroxygruppe der Anhydroglucoseeinheit durch $C_1$-$C_4$-Alkyl, insbesondere Methyl, und an ein bis zwei sekundären Hydroxygruppen durch Hydroxy-$C_2$-$C_4$-alkyl, z.B. 2-Hydroxyäthyl oder 2- oder 3-Hydroxypropyl, veräthert ist, z.B. 2- oder 3-Hydroxypropylmethyl-β-cyclodextrin oder 2-Hydroxyäthyl-methyl-β-cyclodextrin.

Bevorzugt sind die ausschliesslich durch Hydroxy-$C_2$-$C_4$-alkyl substituierten β-Cyclodextrinderivate, z.B. 2- oder 3-Hydroxypropyl-β-cyclodextrin oder 2-Hydroxyäthyl-β-cyclodextrin.

β-Cyclodextrin selbst ist eine bekannte zyklische Verbindung mit 7 Anhydroglucoseeinheiten (Cycloheptaamylose). Jede Anhydroglucoseeinheit ist in 2-, 3- und 6-Stellung durch Hydroxygruppen substituiert, welche sich nicht vollständig veräthern lassen. Der Grad der Substitution oder Verätherung durch $C_1$-$C_4$-Alkylgruppen wird durch die Kennzahl DS = Durchschnittlicher Substitutionsgrad angegeben. Diese Kennzahl ist ein gemittelter Wert der verätherten Hydroxygruppen pro Anhydroglucoseeinheit. Der DS-Wert von geeigneten Derivaten beträgt ca. 0,05 bis 2,0, vorzugsweise ca. 0,2 bis 1,5. Der Bereich von 0,5 bis 1,2 ist besonders bevorzugt.

Der Grad der Substitution oder Verätherung durch Hydroxy-$C_2$-$C_4$-alkylgruppen wird durch die Kennzahl MS = Molarer Substitutionsgrad angegeben. Diese Kennzahl gibt die Anzahl der Mole des zweiten Alkylierungsmittels an, z.B. Propylenoxid oder Aethylenoxid, welche bei der Herstellung des verätherten β-Cyclodextrinderivats verwendet worden sind. Wegen der Substitutionsneigung der gebildeten Hydroxygruppe in der Seitenkette bei möglicher weiterer Polymerisation und dem dadurch bedingten erhöhten Verbrauch an Alkylierungsmittel kann der MS-Wert höher als der DS-Wert sein. Der MS-Wert von geeigneten Derivaten beträgt ca. 0,05 bis 10, vorzugsweise ca. 0,2 bis 2. Besonders bevorzugt ist der MS-Wert von ca. 0,25 bis 1.

Die Herstellung der genannten β-Cyclodextrinderivate ist bekannt oder kann in an sich bekannter Weise erfolgen. So können β-Cyclodextrinderivate welche durch $C_1$-$C_4$-Alkyl und Hydroxy-$C_2$-$C_4$-alkyl substituiert sind, durch Umsetzung von β-Cyclodextrin mit mindestens einem Aequivalent Alkoholat, z.B. Natriummethanolat, in einem aprotischen Lösungsmittel, z.B. Dimethylformamid, Acetonitril oder Dimethoxyäthan, und anschliessende Umsetzung mit ein bis zwei Aequivalenten des zweiten Alkylierungsmittels, z.B. Aethylen- oder Propylenoxid, hergestellt werden. Bei Herstellung von Hydroxyäthyl-oder Hydroxypropyl-β-cyclodextrin geht man einstufig vor und setzt nur mit Aethylen-oder Propylenoxid um, z.B. nach dem in der U.S. Patentschrift 3,459,731 beschriebenen Verfahren oder analog der Methode von J. Szejtli et al., Stärke 32, 165 (1980) oder gem. der Methode nach A.P. Croft und R.A. Bartsch, Tetrahedron 39, 1417 (1983).

Besonders bevorzugt sind 2- oder 3-Hydroxypropyl-β-cyclodextrinderivate mit einem MS-Wert von ca. 0,25-1,0 und einer mittleren Molmasse von ca. 1300-1600.

Diese Derivate sind z.B. bei der Firma Wacker bzw. Consortium für Elektrochemische Industrie, D-8000 München, kommerziell erhältlich.

Die Trägerflüssigkeit ist eine sterile, pyrogenfreie, wässrige Lösung, welche den Wirkstoff Carbamazepin in therapeutisch wirksamer Dosis, das weiter vorn beschriebene $\beta$-Cyclodextrinderivat als Löslichkeitsvertmittler und gegebenenfalls auch weitere wasserlösliche Hilfsstoffe enthält, z.B. für die Herstellung von Trockenpräparaten wie Lyophilisaten oder Eindampfrückständen oder fertig zubereiteten wässrigen Lösungen oder Dispersionen geeignete, pharmazeutisch verwendbare Hilfsstoffe.

Für die Herstellung von Trockenpräparaten geeignete wasserlösliche Hilfsstoffe sind z.B. nichtionische isotonische Zusätze - Gerüstbildner - wie Sorbit, Mannit oder Glucose.

Für die Herstellung von fertig zubereiteten wässrigen Lösungen geeignete Hilfsstoffe sind z.B. ionische Zusätze wie Kochsalz oder Glycin. Insbesondere sind die genannten Zusätze in den vorgeschriebenen Mengen enthalten, welche für die Herstellung von isotonischen Bedingungen der intravenösen Lösung erforderlich sind.

Geeignete Hilfsstoffe für intravenöse Lösungen sind ausserdem die bekannten für flüssige pharmazeutische Formulierungen verwendbaren, Netzmittel oder Tenside, insbesondere nichtionische Tenside vom Typ Fettsäure-Polyhydroxyalkoholester wie Sorbitanmonolaurat, -oleat, -stearat oder -palmitat, Sorbitantristearat oder -trioleat, Polyoxyäthylen-Addukte von Fettsäure-Polyhydroxyalkoholestern wie Polyoxyäthylen-sorbitanmonolaurat, -oleat, -stearat, -palmitat, -tristearat oder -trioleat, Polyäthylenglycol-Fettsäureester wie Polyoxyäthylstearat, Polyäthylenglycol-400-stearat, Polyäthylenglycol-2000-stearat, insbesondere Aethylenoxid-Propylenoxid Blockpolymere vom Typ Pluronic® (Wyandotte Chem. Corp.) oder Synperonic® (ICI) sowie Polyäthylenglycoläther-Addukte, z.B. Tetrahydrofurylalkoholpolyäthylenglycoläther.

Die genannten Tenside können in Gewichtsmengenverhältnissen von Wirkstoff zu Tensid von ca. 1:0,001 bis 1:0,1, vorzugsweise von ca. 1:0,03 bis 1:0,1, in der parenteralen Lösung vorhanden sein.

Die vorliegende Erfindung betrifft vorzugsweise eine pharmazeutische Zusammensetzung für die intravenöse Applikation von Carbamazepin enthaltend

a) Carbamazepin;

b) als Löslichkeitsvermittler durch 2- oder 3-Hydroxypropyl oder 2-Hydroxyäthyl veräthertes $\beta$-Cyclodextrin und

c) die wässrige Trägerflüssigkeit und gegebenenfalls weitere pharmazeutische Hilfsstoffe.

Die vorliegende Erfindung betrifft vor allem eine pharmazeutische Zusammensetzung für die intravenöse Applikation von Carbamazepin in Form einer wässrigen Lösung enthaltend

a) Carbamazepin;

b) als Löslichkeitsvermittler durch 2- oder 3-Hydroxypropyl veräthertes $\beta$-Cyclodextrin (MS = ca. 0,25 bis 1; Molmasse ca. 1300-1600) und

c) die wässrige Trägerflüssigkeit und gegebenenfalls weitere pharmazeutische Hilfsstoffe.

Die Erfindung betrifft ausserdem die Einschlussverbindung von Carbamazepin in einem durch $C_1$-$C_4$-Alkyl und/oder Hydroxy-$C_2$-$C_4$-alkyl verätherten $\beta$-Cyclodextrinderivat, wobei die Hydroxygruppe sich in einer höheren als der 1-Stellung des $C_2$-$C_4$-Alkylrests befindet, insbesondere in 2- oder 3-Hydroxypropyl-$\beta$-cyclodextrin.

Die Erfindung betrifft ausserdem ein Trockenpräparat, z.B. einen Eindampfrückstand, insbesondere ein Lyophilisat, bestehend aus der Einschlussverbindung von Carbamazepin in einem durch $C_1$-$C_4$-Alkyl und/oder Hydroxy-$C_2$-$C_4$-alkyl verätherten $\beta$-Cyclodextrinderivat, wobei die Hydroxygruppe sich in einer höheren als der 1-Stellung des $C_2$-$C_4$-Alkylrests befindet, und für die Herstellung von Trockenpräparaten geeigneten Hilfsstoffen, insbesondere das Trockenpräparat bestehend aus der Einschlussverbindung von Carbamazepin in 2- oder 3-Hydroxypropyl-$\beta$-cyclodextrin.

Die vorliegende Erfindung betrifft ebenfalls ein Verfahren zur Herstellung einer pharmazeutischen Zusammensetzung für die intravenöse Applikation von Carbamazepin, welches dadurch gekennzeichnet ist, dass man

$\alpha$) als Löslichkeitsvermittler durch $C_1$-$C_4$-Alkyl und/oder Hydroxy-$C_2$-$C_4$-alkyl, wobei die Hydroxygruppe sich in einer höheren als der 1-Stellung des $C_2$-$C_4$-Alkylrests befindet, veräthertes $\beta$-Cyclodextrin in der wässrigen Trägerflüssigkeit löst, Carbamazepin und gegebenenfalls weitere wasserlösliche pharmazeutische Hilfsstoffe zur Trägerflüssigkeit zusetzt und gegebenenfalls die Lösung homogenisiert; oder

$\beta$) ein Trockenpräparat enthaltend den Wirkstoff Carbamazepin und durch $C_1$-$C_4$-Alkyl und/oder Hydroxy-$C_2$-$C_4$-alkyl, wobei die Hydroxygruppe sich in einer höheren als der 1-Stellung des $C_2$-$C_4$-Alkylrests befindet, veräthertes $\beta$-Cyclodextrin herstellt und gegebenenfalls für die Herstellung von Trockenpräparaten geeignete wasserlösliche Hilfsstoffe hinzufügt und das Trockenpräparat in der wässrigen Trägerflüssigkeit auflöst.

4

Verfahren α

Nach dieser Variante wird das pyrogenfreie und gegebenenfalls sterilfiltrierte Wasser vorgelegt und gegebenenfalls unter Erwärmen, z.B. in einem Temperaturbereich von etwa 20° bis etwa 100° C, vorzugsweise 40-60° C, der Löslichkeitsvermittler, insbesondere das 2- oder 3-Hydroxypropyl-β-cyclodextrin, sowie die weiteren Hilfsstoffe zugegeben. Zu dieser Grundlage wird anschliessend der Wirkstoff, insbesondere gemahlenes, wasserfreies Carbamazepin, gegeben. Der Lösungsvorgang kann durch abschliessendes Homogenisieren, z.B. durch Behandeln mit Ultraschall oder hochtouriges Rühren, z.B. mit einem Schnellrührer der Fa. Vortex, beschleunigt bzw. vervollständigt werden. Es wird eine klare Lösung erhalten, die keinerlei Aggregate enthält und homogen ist. So wird keine Lichtstreuung im Sinne eines Tyndalleffekts beobachtet.

Die Lösung lässt sich je nach Wirkstoffgehalt in Ampullen oder Injektionsfläschchen mit Volumina von ca. 1 ml bis ca. 50 ml abfüllen, die dann gegebenenfalls im Autoklaven hitzesterilisiert (120° C/20 min) werden, oder sie wird sterilfiltriert und unter aseptischen Bedingungen abgefüllt.


Verfahren β

Das erfindungsgemässe Trockenpräparat wird hergestellt, indem man z.B. die für die intravenöse Applikation vorgesehene Menge des Carbamazepins in mikronisierter Form in einem Suspensionsmedium, z.B. pyrogenfreiem Wasser, welches den Löslichkeitsvermittler und gegebenenfalls die pharmazeutisch annehmbaren Zusatzstoffe (Hilfsstoffe) enthält, suspendiert und das Lösungsmittel entfernt.

Das Trockenpräparat kann wasserlösliche Zusätze oder Hilfsstoffe wie Kochsalz, Mannit oder Glucose enthalten, welche z.B. zur Herstellung von isotonischen Bedingungen erforderlich sind. Nach dem Auflösen der Zusatzstoffe in Wasser zur Injektion wird Carbamazepin zugegeben und gelöst. Nach Sterilfiltration kann die Herstellung des Trockenpräparats durch Eindampfen oder mittels bekannter Gefriertrocknungsmethoden erfolgen, z.B. indem man eine bestimmte Menge der hergestellten Suspension üblicherweise in geeignete Behälter, wie Ampullen, z.B. Glasstechampullen (Vials), abfüllt und die abgefüllten Stechampullen bei ca. -40° bis -50° C einfriert und anschliessend bei einem Druck von ca. 0,2-0,6 mbar durch langsames Erwärmen in einem Zeitraum von ca. 24-48 h bis zu einer Endtemperatur von ca. 25° -35° C lyophilisiert.

Ueberraschenderweise gelingt es mit den genannten Verfahren Trockenpräparate, insbesondere Lyophilisate, und daraus rekonstituierbare Lösungen herzustellen, welche stabil und zur Injektion geeignet sind.

Die Verwendung des genannten $C_1$-$C_4$-Alkyl und/oder Hydroxy-$C_2$-$C_4$-alkyl-β-cyclodextrin-Aetherderivates in einer der beiden Verfahrensvarianten ist ebenfalls Gegenstand der vorliegenden Erfindung.

Die Verwendung des gemäss den genannten Verfahren erhältlichen Trockenpräparates zur Herstellung von Infusions- und Injektionslösungen ist ebenfalls Gegenstand der vorliegenden Erfindung. Die Infusions- und Injektionslösungen können intravenös appliziert werden.

Das erfindungsgemäss erhältliche Trockenpräparat wird vorzugsweise unmittelbar vor der Applikation als klare Lösung in der für die Infusion oder Injektion vorgesehenen Flüssigkeitsmenge, insbesondere keimfreiem, pyrogenfreiem Wasser zur Injektion, rekonstituiert. Durch Aufschütteln bildet sich wieder die homogene Lösung des Wirkstoffs. Statt eines Trockenpräparats enthaltend Carbamazepin und sämtliche übrigen Zusatzstoffe wie Kochsalz oder Mannit lässt sich auch ein Trockenpräparat enthaltend nur Carbamazepin und den Löslichkeitsvermittler b) - ohne Zusatzstoffe c) - lösen. Diese Variante eignet sich zur Herstellung von Infusionslösungen.

Die pharmazeutischen Zusammensetzungen zeichnen sich durch gute Lagerstabilität aus. Es wird keine Abscheidung von Niederschlägen oder Kristallen aus der Lösung beobachtet.

In einer besonders bevorzugten Ausführungsform werden Injektions- und Infusionslösungen, die die üblichen Dosen von 10-250 mg Carbamazepin enthalten, mit einem Gesamtvolumen von 1,0-50 ml, insbesondere 2,0-10,0 ml, hergestellt. Diese Lösungen können als "ready to use" Präparate verwendet werden.

Die erfindungsgemässen intravenös applizierbaren Darreichungsformen besitzen wertvolle pharmakologische Eigenschaften und können als Antineuralgika und Antikonvulsiva zur Behandlung von starken Krampfzuständen und Konvulsionen, insbesondere Konvulsionen verschiedenartiger Genese, z.B. Behandlung der Epilepsie, sowie zum prophylaktischen Schutz prädisponierter Personen gegen solche plötzlich auftretende Konvulsionen in Situationen, wobei die sonst übliche perorale Verabreichung nicht erfolgen kann, etwa bei Unfällen oder während Operationen, verwendet werden. Die Verwendung dieser Darreichungsform zur Behandlung solcher Krankheiten, insbesondere der Epilepsie, ist ebenfalls Gegenstand der vorliegenden Erfindung.

Die Erfindung betrifft insbesondere auch die in den Beispielen beschriebenen Zubereitungen bzw. Herstellungsverfahren.

Die nachfolgenden Beispiele dienen lediglich der Erläuterung der vorstehend beschriebenen Erfindung; sie sollen diese jedoch in ihrem Umfang in keiner Weise einschränken.

Beispiele 1-10: Injektionslösungen

Beispiel 1:

Zur Herstellung einer Carbamazepin enthaltenden Injektionslösung werden 100,0 g 2- oder 3-Hydroxypropyl-$\beta$-cyclodextrin (MS = 0,6) und 18,0 g Glucose in 80 g pyrogenfreiem, bidestilliertem Wasser gelöst. Der pH-Wert der Lösung wird durch Zusatz der notwendigen Menge 0.1-normaler Natronlauge oder 0,1-normaler Salzsäure auf 6.5 eingestellt. In dieser Lösung werden 3,0 g fein gemahlenes Carbamazepin durch Erwärmen auf 50°C und Rühren gelöst. Nach Abkühlen auf Raumtemperatur wird mit pyrogenfreiem, bidestilliertem Wasser auf ein Gesamtvolumen von 950 ml ergänzt. Nach Prüfung des pH-Werts und gegebenenfalls Korrektur mittels 0,1 normaler Natronlauge oder 0,1 normaler Salzsäure wird auf das Endvolumen von 1000 ml aufgefüllt. Diese Lösung wird filtriert, in Ampullen zu je 10 ml abgefüllt und in einem Autoklaven 20 Minuten bei 120°C sterilisiert.
Die Lösung hat folgende Eigenschaften:
Gehalt: 30 mg Carbamazepin in 10,0 ml
Aussehen: klar, farblos bis schwach gelbstichig
pH-Wert: 6,5-7,0
Osmolalität: 300 mOsm

Beispiel 2:

In ähnlicher Weise wie in Beispiel 1 werden 1000 ml einer Lösung aus 100,0 g 2- oder 3-Hydroxypropyl-$\beta$-cyclodextrin (MS = 0,6), 18,0 g Mannit und 3,0 g Carbamazepin in pyrogenfreiem, bidestilliertem Wasser hergestellt.
Die Lösung hat folgende Eigenschaften:
Gehalt: 30 mg Carbamazepin in 10,0 ml
Aussehen: klar, farblos bis schwach gelbstichig
pH-Wert: 6,5-7,0
Osmolalität: 300 mOsm

Beispiel 3:

In ähnlicher Weise wie in Beispiel 1 werden 1000 ml einer Lösung aus 100,0 g 2- oder 3-Hydroxypropyl-$\beta$-cyclodextrin (MS = 0,6), 3,5 g Natriumchlorid und 3,0 g Carbamazepin in pyrogenfreiem, bidestilliertem Wasser hergestellt.
Die Lösung hat folgende Eigenschaften:
Gehalt: 30 mg Carbamazepin in 10,0 ml
Aussehen: klar, farblos bis schwach gelbstichig
pH-Wert: 6,5-7,0
Osmolalität: 300 mOsm

Beispiel 4:

In ähnlicher Weise wie in Beispiel 1 werden 1000 ml einer Lösung aus 150,0 g 2- oder 3-Hydroxypropyl-$\beta$-cyclodextrin (MS = 0,6), 5,2 g Glucose und 5,0 g Carbamazepin in pyrogenfreiem, bidestilliertem Wasser hergestellt.
Die Lösung hat folgende Eigenschaften:
Gehalt: 50 mg Carbamazepin in 10,0 ml

Aussehen: klar, farblos bis schwach gelbstichig
pH-Wert: 6,4-7,0
Osmolalität: 290 mOsm

Beispiel 5:

In ähnlicher Weise wie in Beispiel 1 werden 1000 ml einer Lösung aus 150,0 g 2- oder 3-Hydroxypropyl-$\beta$-cyclodextrin (MS = 0,6), 0,9 g Natriumchlorid und 5,0 g Carbamazepin in pyrogenfreiem, bidestilliertem Wasser hergestellt.
Die Lösung hat folgende Eigenschaften:
Gehalt: 50 mg Carbamazepin in 10,0 ml
Aussehen: klar, farblos bis schwach gelbstichig
pH-Wert: 6,4-7,0
Osmolalität: 290 mOsm

Beispiel 6:

In ähnlicher Weise wie in Beispiel 1 werden 1000 ml einer Lösung aus 200,0 g 2- oder 3-Hydroxypropyl-$\beta$-cyclodextrin (MS = 0,6) und 10,0 g Carbamazepin in pyrogenfreiem, bidestilliertem Wasser hergestellt.
Die Lösung hat folgende Eigenschaften:
Gehalt: 100 mg Carbamazepin in 10,0 ml
Aussehen: klar, farblos bis schwach gelbstichig
pH-Wert: 6,4-7,0
Osmolalität: 340 mOsm

Beispiel 7:

In ähnlicher Weise wie in Beispiel 1 werden 1000 ml einer Lösung aus 100,0 g 2- oder 3-Hydroxypropyl-$\beta$-cyclodextrin (MS = 0,9), 21,0 g Glucose und 3,0 g Carbamazepin in pyrogenfreiem, bidestilliertem Wasser hergestellt.
Die Lösung hat folgende Eigenschaften:
Gehalt: 30 mg Carbamazepin in 10,0 ml
Aussehen: klar, farblos bis schwach gelbstichig
pH-Wert: 6,5-7,0
Osmolalität: 290 mOsm

Beispiel 8:

In ähnlicher Weise wie in Beispiel 1 werden 1000 ml einer Lösung aus 100,0 g 2- oder 3-Hydroxypropyl-$\beta$-cyclodextrin (MS = 0,9), 4,4 g Natriumchlorid und 3,0 g Carbamazepin in pyrogenfreiem, bidestilliertem Wasser hergestellt.
Die Lösung hat folgende Eigenschaften:
Gehalt: 30 mg Carbamazepin in 10,0 ml
Aussehen: klar, farblos bis schwach gelbstichig
pH-Wert: 6,5-7,0
Osmolalität: 290 mOsm

Beispiel 9:

In ähnlicher Weise wie in Beispiel 1 werden 1000 ml einer Lösung aus 150,0 g 2- oder 3-Hydroxypropyl-$\beta$-cyclodextrin (MS = 0,9), 14,8 g Mannit und 5,0 g Carbamazepin in pyrogenfreiem, bidestilliertem Wasser hergestellt.

7

EP 0 400 609 A1

Die Lösung hat folgende Eigenschaften:
Gehalt: 50 mg Carbamazepin in 10,0 ml
Aussehen: klar, farblos bis schwach gelbstichig
pH-Wert: 6,5-7,0
Osmolalität: 290 mOsm


Beispiel 10:

In ähnlicher Weise wie in Beispiel 1 werden 1000 ml einer Lösung aus 150,0 g 2- oder 3-Hydroxypropyl-$\beta$-cyclodextrin (MS = 0,9), 10,0 g Carbamazepin in pyrogenfreiem, bidestilliertem Wasser hergestellt.
Die Lösung hat folgende Eigenschaften:
Gehalt: 100 mg Carbamazepin in 10,0 ml
Aussehen: klar, farblos bis schwach gelbstichig
pH-Wert: 6,5-7,0
Osmolalität: 280 mOsm


Beispiele 11-12: Lyophilisate geeignet für die intravenöse Anwendung


Beispiel 11:

Zur Herstellung eines Carbamazepin enthaltenden Lyophilisats verwendbar für die Herstellung von intravenösen Lösungen werden 150,0 g 2- oder 3-Hydroxypropyl-$\beta$-cyclodextrin (MS = 0,6) und 5,2 g Mannit in 80 g pyrogenfreiem, bidestilliertem Wasser gelöst. Der pH-Wert der Lösung wird durch Zusatz der notwendigen Menge 0,1-normaler Natronlauge oder 0,1-normaler Salzsäure auf ca. 6,5 eingestellt. In dieser Lösung werden 5,0 g fein gemahlenes Carbamazepin durch Erwärmen auf 50°C und Rühren gelöst. Nach Abkühlen auf Raumtemperatur wird mit pyrogenfreiem, bidestilliertem Wasser auf ein Gesamtvolumen von 450 ml ergänzt. Nach Prüfung des pH-Werts und gegebenenfalls Korrektur mittels 0,1-normaler Natronlauge oder 0,1-normaler Salzsäure wird auf das Endvolumen von 500,0 ml aufgefüllt. Diese Lösung wird sterilfiltriert und in Portionen zu je 5,0 ml in 10-ml-Ampullen abgefüllt. Die abgefüllten Ampullen werden in einer Gefriertrocknungsapparatur bei -40°C eingefroren, bei einem Druck von 0,5 mbar und einer Endtemperatur von +35°C lyophilisiert und danach verschlossen.
Zur Rekonstitution der gebrauchsfertigen Lösung werden mit einer Injektionsspritze 10,0 ml steriles Wasser zur Injektion zu einer Ampulle mit Trockensubstanz gegeben. Nach kurzem Umschwenken entsteht wieder eine homogene Lösung, die folgende Eigenschaften hat:
Gehalt: 50 mg Carbamazepin in 10,0 ml
Aussehen: klar, farblos bis schwach gelbstichig
pH-Wert: 6,5-7,0
Osmolalität: 300 mOsm


Beispiel 12:

In ähnlicher Weise wie in Beispiel 11 wird ein Carbamazepin enthaltendes Lyophilisat zur Injektion hergestellt und zwar durch Lösen von 200,0 g Hydroxypropyl-$\beta$-cyclodextrin (MS = 0,9) in 250 g pyrogenfreiem, bidestilliertem Wasser, Einstellen des pH-Wertes der Lösung auf ca. 6,5, Auflösen von 10,0g fein gemählenem Carbamazepin in dieser Lösung durch Rühren und Erwärmen auf 50°C, Ergänzen auf ein Gesamt volumen von 500 ml mit pyrogenfreiem bidestilliertem Wasser, Sterilfiltration, Abfüllen in Ampullen, Einfrieren und Lyophilisieren.
Zur Rekonstitution der gebrauchsfertigen Lösung werden mit einer Injektionsspritze zu einer Ampulle mit Trockensubstanz 10,0 ml steriles Wasser zur Injektion gegeben. Nach kurzem Umschwenken entsteht wieder eine homogene Lösung, die folgende Eigenschaften hat:
Gehalt: 100 mg Carbamazepin in 10,0 ml
Aussehen: klar, schwach gelbstichig
pH-Wert: 6,5-7,0

8

Osmolalität: 280 mOsm

Beispiel 13:

Vorbereitung einer Lösung von Carbamazepin mit Hilfe einer 45 % (G/V) wässrigen Lösung von 2- oder 3-Hydroxypropyl-$\beta$-cyclodextrin (MS = 0,9). 45 g 2- oder 3-Hydroxypropyl-$\beta$-cyclodextrin werden portionenweise unter Rühren in destilliertem Wasser gelöst und das Endvolumen auf 100 ml eingestellt. 20 mg Carbamazepin werden mit 1 ml dieser wässrigen Lösung vermischt und solange mit Ultraschall behandelt, bis eine klare Lösung entsteht (1 min., Stufe 20 auf der Skala eines Sonifier Apparates). Diese Lösung ist während mehrerer Wochen stabil, d.h. sie bleibt klar (keine Bildung eines Niederschlages) unter Erhalt der pharmakologischen Wirkung. Zur Vorbereitung von Verdünnungsreihen für in-vivo Versuche wird diese Stammlösung mit beliebigen Mengen Wasser verdünnt, ohne dass Carbamazepin aus der Lösung ausfällt.

Beispiel 14: Vorbereitung steriler Lösungen.

Lösungen, hergestellt nach Beispiel 1, werden während 3 Minuten bei 120 °C erhitzt, anschliessend auf 4 °C abgekühlt und bei dieser Temperatur aufbewahrt.

Beispiel 15: Herstellung eines gefriergetrockneten Pulvers.

Nach Beispiel 13 hergestellte Lösungen werden bei -80 °C während mehrerer Stunden eingefroren und das Wasser bei 0,0133 mbar durch Sublimieren entfernt. Der Rückstand besteht aus einem weissen, nicht hygroskopischen und stabilen Pulver, das sich direkt in Wasser rasch und vollständig löst.

Beispiel 16: Herstellung eines Eindampfrückstandes.

Nach Beispiel 13 hergestellte Lösungen werden in einer Vakuumzentrifuge bei Zimmer temperatur bei 0,1-1,33 mbar eingedampft. Der weisse Eindampfrückstand ist nicht hygroskopisch, längere Zeit haltbar und löst sich direkt in Wasser.

Beispiel 17: Herstellung eines Eindampfrückstandes.

Nach Beispiel 13 hergestellte Lösungen werden bei ca. 15,96 mbar und 40 °C eingedampft. Der Rückstand ist während mehrerer Wochen stabil und löst sich direkt in Wasser.

Beispiel 18: Herstellung wässriger Lösungen.

Die unter den Nummern 14 bis 17 hergestellten Trockenpräparate lösen sich ohne Hilfsmittel in weniger als einer Minute bei 20 °C in Wasser, während sich mikrokristallisiertes Carbamazepin unter den gleichen Bedingungen nicht löst.

Beispiel 19:

Die akute Schutzwirkung von Carbamazepin nach intravenöser Verabreichung im Vergleich zur oralen wurde mit Hilfe des maximalen Elektroschocks ermittelt. Die folgenden Methoden wurden verwendet:
- Elektroschock Test an Ratten:
Tonische Konvulsionen der Kinterextremitäten wurden durch Verabreichung eines Wechselstroms von 50 Hz und 36 mA während 0,63 Sekunden mit Hilfe von Cornealelektroden ausgelöst. Es wurden 10 Ratten pro Dosis und Zeiteinheit verwendet; eine Gruppe diente als Kontrolle. Die Anzahl der Ratten, die keine tonische Konvulsion der Hinterextremitäten aufwiesen, wurde für jede Gruppe bestimmt. Die $ED_{50}$ wurde mit Hilfe der Regressionsanalyse nach der Methode von Spearman und Kärber berechnet.

- Elektroschock Test an Mäusen:

Tonische Konvulsionen der Hinterextremitäten wurden durch Verabreichung eines Wechselstromes von 50 Hz und 18 mA während 0,2 Sekunden mittels Cornealelektroden ausgelöst. Für jede Dosis und Zeiteinheit wurden 10 Mäuse verwendet; eine Gruppe diente als Kontrolle. Die Anzahl der Mäuse, die keine tonische Konvulsion der Hinterextremitäten zeigten, wurde für jede Gruppe bestimmt. Die $ED_{50}$ wurde mit Hilfe der Regressionsanalyse nach der Methode von Spearman und Kärber bestimmt.

Die pharmakologischen Eigenschaften der verschiedenen Lösungen sind in den Tabellen 1 bis 3 dargestellt. Die antikonvulsive Wirkung von Carbamazepin tritt nach der intravenösen schneller als nach der oralen Applikation auf (Tabelle 3). Ueberraschenderweise sind die $ED_{50}$ nach intravenöser Verabreichung wesentlich kleiner als nach oraler Verabreichung (Tabelle 3).

Tabelle 1:

| Lösung von 20 mg Carbamazepin pro ml Hydroxypropyl-$\beta$-cyclodextrin/Wasser 16,6 % (G/V). | Schutz vor dem maximalen Elektroschock in der Maus ($ED_{50}$). |
|---|---|
| Lösung nach Beispiel 13 | 6,6 mg/kg |
| Lösung nach Beispiel 14 | 5,7 mg/kg |
| Lösung nach Beispiel 16 | 6,6 mg/kg |
| Lösung nach Beispiel 15 | 6,6 mg/kg |
| Lösung nach Beispiel 17 | 6,2 mg/kg |
| Lösung von Hydroxypropyl-$\beta$-dextrin | keine $ED_{50}$ ermittelbar |
| Carbamazepin wurde intravenös 30 Minuten vor dem Elektroschock injiziert. Die $ED_{50}$-Werte wurden durch Regressionsanalyse aus drei verschiedenen Dosen (10 Mäuse pro Dosis) berechnet. | |

Tabelle 2:

| Zeitverlauf der Schutzwirkung von Carbamazepin nach intravenöser Verabreichung bei Mäusen und Ratten ($ED_{50}$) | | |
|---|---|---|
| Zeit in min | $ED_{50}$ in mg/kg in | |
| | Mäuse | Ratten |
| 2,5 | 5,4 | - |
| 5 | 6,2 | 1,6 |
| 15 | 5,4 | 2,5 |
| 30 | 6,2 | 3,0 |
| 60 | 9,3 | - |
| 120 | 15,8 | 4,0 |
| 240 | $\gg$20 | 40 % bei 10 mg/kg |
| Die $ED_{50}$-Werte werden durch Regressionsanalyse aus drei verschiedenen Dosen (10 Tiere pro Dosis) berechnet. | | |

Tabelle 3:

| Vergleich der Schutzwirkung von Carbamazepin nach intravenöser und oraler Verabreichung (ED$_{50}$) | | | | |
|---|---|---|---|---|
| Zeit in min | ED$_{50}$ in mg/kg | | | |
| | intravenös | | oral | |
| | Mäuse | Ratten | Mäuse | Ratten |
| 2,5 | 5,4 | 1,9 | 10 % bei 60 | ≫60 |
| 5,0 | 6,2 | 1,6 | 22,7 | 60,0 |
| 15 | 5,4 | 2,5 | 22,3 | 15,4 |
| 30 | 6,2 | 3,0 | 10,7 | 9,6 |
| 60 | 9,3 | 2,5 | 13,0 | 10,0 |
| 120 | 15,8 | 4,0 | 27,0 | 12,5 |
| Die ED$_{50}$-Werte werden durch Regressionsanalyse aus drei verschiedenen Dosen (10 Tiere pro Dosis) berechnet. | | | | |

Beispiel 20: Herstellung von Ampullen

4,5 g Hydroxypropyl-$\beta$-cyclodextrin (MS = 0,9) und 150 mg Carbamazepin werden unter Rühren mit Ultraschall innerhalb 2 Minuten in 10 ml Wasser gelöst. Diese Lösung wird durch ein Membranfilter (0,22 Mikrometer) filtriert, die klare Lösung in Ampullen gefüllt und durch 3-minütiges Erhitzen bei 120°C sterilisiert.

## Ansprüche

1. Pharmazeutische Zusammensetzung für die intravenöse Applikation von Carbamazepin enthaltend:
   a) Carbamazepin;
   b) als Löslichkeitsvermittler durch $C_1$-$C_4$-Alkyl und/oder Hydroxy-$C_2$-$C_4$-alkyl veräthertes $\beta$-Cyclodextrin, wobei die Hydroxygruppe sich in einer höheren als der 1-Stellung des $C_2$-$C_4$-Alkylrests befindet, und
   c) die wässrige Trägerflüssigkeit und gegebenenfalls weitere wasserlösliche pharmazeutische Hilfsstoffe.

2. Pharmazeutische Zusammensetzung gemäss Anspruch 1 enthaltend:
   a) Carbamazepin;
   b) als Löslichkeitsvermittler durch 2- oder 3-Hydroxypropyl oder 2-Hydroxyäthyl veräthertes $\beta$-Cyclodextrin und
   c) die wässrige Trägerflüssigkeit und weitere wasserlösliche pharmazeutische Hilfsstoffe.

3. Pharmazeutische Zusammensetzung gemäss Anspruch 1 enthaltend:
   a) Carbamazepin;
   b) als Löslichkeitsvermittler 2- oder 3-Hydroxypropyl-$\beta$-cyclodextrin (MS = ca. 0,25 bis 1; Molmasse 1300-1600) und
   c) die wässrige Trägerflüssigkeit und weitere wasserlösliche pharmazeutische Hilfsstoffe.

4. Pharmazeutische Zusammensetzung gemäss Anspruch 1 in Form einer Injektions- oder Infusionslösung.

5. Trockenpräparat bestehend aus der Einschlussverbindung von Carbamazepin in einem durch $C_1$-$C_4$-Alkyl und/oder Hydroxy-$C_2$-$C_4$-alkyl verätherten $\beta$-Cyclodextrinderivat und gegebenenfalls für die Herstellung von Trockenpräparaten geeigneten wasserlöslichen Hilfsstoffen.

6. Trockenpräparat gemäss Anspruch 5 bestehend aus der Einschlussverbindung von Carbamazepin in 2-oder 3-Hydroxypropyl-$\beta$-cyclodextrin.

7. Die Einschlussverbindung von Carbamazepin in einem durch $C_1$-$C_4$-Alkyl und/oder Hydroxy-$C_2$-$C_4$-

11

alkyl verätherten $\beta$-Cyclodextrinderivat, wobei die Hydroxygruppe sich in einer höheren als der 1-Stellung des $C_2$-$C_4$-Alkylrests befindet.

8. Die Einschlussverbindung gemäss Anspruch 7 bestehend aus Carbamazepin in 2- oder 3-Hydroxypropyl-$\beta$-cyclodextrin.

9. Verfahren zur Herstellung einer pharmazeutischen Zusammensetzung gemäss Anspruch 1, dadurch gekennzeichnet dass man

$\alpha$) als Löslichkeitsvermittler durch $C_1$-$C_4$-Alkyl und/oder Hydroxy-$C_2$-$C_4$-alkyl, wobei die Hydroxygruppe sich in einer höheren als der 1-Stellung des $C_2$-$C_4$-Alkylrests befindet, veräthertes $\beta$-Cyclodextrin in der wässrigen Trägerflüssigkeit löst, Carbamazepin und gegebenenfalls weitere wasserlösliche pharmazeutische Hilfsstoffe zur Trägerflüssigkeit zusetzt und gegebenenfalls die Lösung homogenisiert; oder

$\beta$) ein Trockenpräparat enthaltend den Wirkstoff Carbamazepin und durch $C_1$-$C_4$-Alkyl und/oder Hydroxy-$C_2$-$C_4$-alkyl, wobei die Hydroxygruppe sich in einer höheren als der 1-Stellung des $C_2$-$C_4$-Alkylrests befindet, veräthertes $\beta$-Cyclodextrin herstellt und gegebenenfalls für die Herstellung von Trockenpräparaten geeignete wasserlösliche Hilfsstoffe hinzufügt und das Trockenpräparat in der Trägerflüssigkeit auflöst.

10. Verwendung von einem durch $C_1$-$C_4$-Alkyl und/oder Hydroxy-$C_2$-$C_4$-alkyl verätherten $\beta$-Cyclodextrinderivat zur Herstellung einer wässrigen, intravenös applizierbaren Lösung von Carbamazepin.

11. Verwendung gemäss Anspruch 10 von 2-oder 3-Hydroxypropyl-$\beta$-cyclodextrin zur Herstellung einer wässrigen, intravenös applizierbaren Lösung von Carbamazepin.

12. Stabile intravenöse Lösungen von Carbamazepin zur Anwendung in einem Verfahren zur therapeutischen Behandlung des menschlichen oder tierischen Körpers.

13. Stabile intravenöse Lösungen von Carbamazepin zur Anwendung als Antineuralgikum und/oder Antikonvulsivum.

Patentansprüche für folgende Vertragsstaaten: ES, GR

1. Verfahren zur Herstellung einer pharmazeutischen Zusammensetzung für die intravenöse Applikation von Carbamazepin enthaltend:

a) Carbamazepin;

b) als Löslichkeitsvermittler durch $C_1$-$C_4$-Alkyl und/oder Hydroxy-$C_2$-$C_4$-alkyl veräthertes $\beta$-Cyclodextrin, wobei die Hydroxygruppe sich in einer höheren als der 1-Stellung des $C_2$-$C_4$-Alkylrests befindet, und

c) die wässrige Trägerflüssigkeit und gegebenenfalls weitere wasserlösliche pharmazeutische Hilfsstoffe, dadurch gekennzeichnet, dass man

$\alpha$) als Löslichkeitsvermittler durch $C_1$-$C_4$-Alkyl und/oder Hydroxy-$C_2$-$C_4$-alkyl, wobei die Hydroxygruppe sich in einer höheren als der 1-Stellung des $C_2$-$C_4$-Alkylrests befindet, veräthertes $\beta$-Cyclodextrin in der wässrigen Trägerflüssigkeit löst, Carbamazepin und gegebenenfalls weitere wasserlösliche pharmazeutische Hilfsstoffe zur Trägerflüssigkeit zusetzt und gegebenenfalls die Lösung homogenisiert; oder

$\beta$) ein Trockenpräparat enthaltend den Wirkstoff Carbamazepin und durch $C_1$-$C_4$-Alkyl und/oder Hydroxyl-$C_2$-$C_4$-alkyl, wobei die Hydroxygruppe sich in einer höheren als der 1-Stellung des $C_2$-$C_4$-Alkylrests befindet, veräthertes $\beta$-Cyclodextrin herstellt und gegebenenfalls für die Herstellung von Trockenpräparaten geeignete wasserlösliche Hilfsstoffe hinzufügt und das Trockenpräparat in der Trägerflüssigkeit auflöst.

2. Verfahren zur Herstellung einer pharmazeutischen Zusammensetzung gemäss Anspruch 1 enthaltend:

a) Carbamazepin;

b) als Löslichkeitsvermittler durch 2- oder 3-Hydroxypropyl oder 2-Hydroxyäthyl veräthertes $\beta$-Cyclodextrin und

c) die wässrige Trägerflüssigkeit und weitere wasserlösliche pharmazeutische Hilfsstoffe.

3. Verfahren zur Herstellung einer pharmazeutischen Zusammensetzung gemäss Anspruch 1 enthaltend:

a) Carbamazepin;

b) als Löslichkeitsvermittler 2- oder 3-Hydroxypropyl-$\beta$-cyclodextrin (MS = ca. 0,25 bis 1; Molmasse 1300-1600) und

c) die wässrige Trägerflüssigkeit und weitere wasserlösliche pharmazeutische Hilfsstoffe.

4. Verfahren zur Herstellung einer pharmazeutischen Zusammensetzung gemäss Anspruch 1 in Form einer Injektions- oder Infusionslösung.

5. Verfahren zur Herstellung eines Trockenpräparats bestehend aus der Einschlussverbindung von Carbamazepin in einem durch $C_1$-$C_4$-Alkyl und/oder Hydroxy-$C_2$-$C_4$-alkyl verätherten $\beta$-Cyclodextrinderivat

EP 0 400 609 A1

und gegebenenfalls für die Herstellung von Trockenpräparaten geeigneten wasserlöslichen Hilfsstoffen.

6. Verfahren zur Herstellung eines Trockenpräparats gemäss Anspruch 5 bestehend aus der Einschlussverbindung von Carbamazepin in 2- oder 3-Hydroxypropyl-$\beta$-cyclodextrin.

7. Verfahren zur Herstellung einer Einschlussverbindung von Carbamazepin in einem durch $C_1$-$C_4$-Alkyl und/oder Hydroxy-$C_2$-$C_4$-alkyl verätherten $\beta$-Cyclodextrinderivat, wobei die Hydroxygruppe sich in einer höheren als der 1-Stellung des $C_2$-$C_4$-Alkylrests befindet.

8. Verfahren zur Herstellung einer Einschlussverbindung gemäss Anspruch 7 bestehend aus Carbamazepin in 2- oder 3-Hydroxypropyl-$\beta$-cyclodextrin.

9. Verfahren zur Herstellung einer pharmazeutischen Zusammensetzung gemäss Anspruch 1, dadurch gekennzeichnet dass man

$\alpha$) als Löslichkeitsvermittler durch $C_1$-$C_4$-Alkyl und/oder Hydroxy-$C_2$-$C_4$-alkyl, wobei die Hydroxygruppe sich in einer höheren als der 1-Stellung des $C_2$-$C_4$-Alkylrests befindet, veräthertes $\beta$-Cyclodextrin in der wässrigen Trägerflüssigkeit löst, Carbamazepin und gegebenenfalls weitere wasserlösliche pharmazeutische Hilfsstoffe zur Trägerflüssigkeit zusetzt und gegebenenfalls die Lösung homogenisiert; oder

$\beta$) ein Trockenpräparat enthaltend den Wirkstoff Carbamazepin und durch $C_1$-$C_4$-Alkyl und/oder Hydroxy-$C_2$-$C_4$-alkyl, wobei die Hydroxygruppe sich in einer höheren als der 1-Stellung des $C_2$-$C_4$-Alkylrests befindet, veräthertes $\beta$-Cyclodextrin herstellt und gegebenenfalls für die Herstellung von Trockenpräparaten geeignete wasserlösliche Hilfsstoffe hinzufügt und das Trockenpräparat in der Trägerflüssigkeit auflöst.

10. Verwendung von einem durch $C_1$-$C_4$-Alkyl und/oder Hydroxy-$C_2$-$C_4$-alkyl verätherten $\beta$-Cyclodextrinderivat zur Herstellung einer wässrigen, intravenös applizierbaren Lösung von Carbamazepin.

11. Verwendung gemäss Anspruch 10 von 2-oder 3-Hydroxypropyl-$\beta$-cyclodextrin zur Herstellung einer wässrigen, intravenös applizierbaren Lösung von Carbamazepin.

12. Stabile intravenöse Lösungen von Carbamazepin zur Anwendung in einem Verfahren zur therapeutischen Behandlung des menschlichen oder tierischen Körpers.

13. Stabile intravenöse Lösungen von Carbamazepin zur Anwendung als Antineuralgikum und/oder Antikonvulsivum.

13

Europäisches
Patentamt

**EUROPÄISCHER RECHERCHENBERICHT**

Nummer der Anmeldung

EP 90 11 0267

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| D,A | US-A-4 727 064 (J. PITHA)<br>* Ansprüche 1,3,6,9,11,23 * | 1,5,7,9 -10 | A 61 K 31/55<br>A 61 K 9/18<br>A 61 K 47/40<br>A 61 K 47/48 |
| A | EP-A-0 197 571 (JANSSEN)<br>* Ansprüche 1,8-10,15; Seite 5, Zeilen 32-33; Seite 6, Zeilen 1-6; Seite 8, Zeilen 6-9 * | 1,5,7,9 -10 | |

RECHERCHIERTE
SACHGEBIETE (Int. Cl.5)

A 61 K

Der vorliegende Recherchenbericht wurde für alle Patentansprüche erstellt

| Recherchenort | Abschlußdatum der Recherche | Prüfer |
|---|---|---|
| DEN HAAG | 07-09-1990 | SCARPONI U. |